(19) Europäisches Patentamt
European Patent Office
Office européen des brevets

(11) **EP 3 557 273 B1**

(12) **EUROPÄISCHE PATENTSCHRIFT**

(45) Veröffentlichungstag und Bekanntmachung des Hinweises auf die Patenterteilung:
**28.07.2021 Patentblatt 2021/30**

(51) Int Cl.:
*G01R 33/56* *(2006.01)*   *G01R 33/561* *(2006.01)*

(21) Anmeldenummer: **18167497.9**

(22) Anmeldetag: **16.04.2018**

(54) **VERFAHREN UND VORRICHTUNG ZUR REKONSTRUKTION VON KONTRASTEN AUS MAGNETRESONANZAUFNAHMEN**

METHOD AND DEVICE FOR THE RECONSTRUCTION OF CONTRASTS FROM MAGNETIC RESONANCE IMAGING

PROCÉDÉ ET DISPOSITIF DE RECONSTRUCTION DE CONTRASTES À PARTIR D'IMAGERIE PAR RÉSONANCE MAGNÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**23.10.2019 Patentblatt 2019/43**

(73) Patentinhaber: **Siemens Healthcare GmbH**
**91052 Erlangen (DE)**

(72) Erfinder:
• **Carinci, Flavio**
**91052 Erlangen (DE)**
• **Paul, Dominik**
**91088 Bubenreuth (DE)**

(56) Entgegenhaltungen:
DE-B3-102016 219 052   US-A1- 2009 224 756
US-A1- 2014 376 794

• MARQUES J P ET AL: "MP2RAGE, a self bias-field corrected sequence for improved segmentation and T"1-mapping at high field", NEUROIMAGE, ELSEVIER, AMSTERDAM, NL, Bd. 49, Nr. 2, 15. Januar 2010 (2010-01-15), Seiten 1271-1281, XP026796256, ISSN: 1053-8119 [gefunden am 2009-10-09]

**Beschreibung**

Die Erfindung betrifft ein Verfahren und eine Vorrichtung zur Rekonstruktion von Kontrasten aus Magnetresonanzaufnahmen mittels einer Parallelen Aufnahme-Technik ("PAT"), sowie eine entsprechende Steuereinrichtung bzw. ein entsprechendes Magnetresonanztomographiesystem.

In einem Magnetresonanzsystem wird üblicherweise der zu untersuchende Körper mit Hilfe eines Grundfeldmagnetsystems einem relativ hohen Grundfeldmagnetfeld, beispielsweise von 1,5 Tesla, 3 Tesla oder 7 Tesla ausgesetzt. Nach Anlegen des Grundfeldes richten sich Kerne im Untersuchungsobjekt mit einem nicht verschwindenden nuklearen magnetischen Dipolmoment, häufig auch Spin genannt, entlang des Feldes aus. Dieses kollektive Verhalten des Spin-Systems wird mit der makroskopischen "Magnetisierung" beschrieben. Die makroskopische Magnetisierung ist die Vektorsumme aller mikroskopischen magnetischen Momente im Objekt an einem bestimmten Ort. Zusätzlich zu dem Grundfeld wird mit Hilfe eines Gradientensystems ein Magnetfeldgradient angelegt, durch den die Magnetresonanzfrequenz (Larmor-Frequenz) am jeweiligen Ort bestimmt wird. Über ein Hochfrequenz-Sendesystem werden dann mittels geeigneter Antenneneinrichtungen hochfrequente Anregungssignale (HF-Pulse) ausgesendet, was dazu führen soll, dass die Kernspins bestimmter, durch dieses Hochfrequenzfeld resonant (d. h. bei der am jeweiligen Ort vorliegenden Larmor-Frequenz) angeregter Kerne um einen definierten Flipwinkel gegenüber den Magnetfeldlinien des Grundmagnetfelds verkippt werden. Wirkt ein solcher HF-Puls auf Spins, die schon angeregt sind, so können diese in eine andere Winkelstellung umgekippt oder sogar in einen Ausgangszustand parallel zum Grundmagnetfeld zurückgeklappt werden. Bei der Relaxation der angeregten Kernspins werden Hochfrequenzsignale, so genannte Magnetresonanzsignale, resonant abgestrahlt, die mittels geeigneter Empfangsantennen (auch Magnetresonanzspulen oder Empfangsspulen genannt) empfangen, anschließend demoduliert und digitalisiert werden und dann als sogenannte "Rohdaten" weiterverarbeitet werden. Die Akquisition der Magnetresonanzsignale erfolgt im Ortsfrequenzraum, dem sogenannten "k-Raum", wobei während einer Messung z. B. einer Schicht der k-Raum entlang einer durch die Schaltung der Gradientenpulse definierten "Gradiententrajektorie" (auch "k-Raum-Trajektorie" genannt) zeitlich durchlaufen wird. Außerdem müssen zeitlich passend koordiniert die HF-Pulse ausgesandt werden. Aus den so akquirierten Rohdaten können nach weiterer Verarbeitungsschritten, die in der Regel auch vom Akquisitionsverfahren abhängen, schließlich mittels einer zweidimensionalen Fourier-Transformation die gewünschten Bilddaten rekonstruiert werden. Alternativ können inzwischen auch dreidimensionale Volumen definiert angeregt und ausgelesen werden, wobei die Rohdaten wiederum nach weiterer Verarbeitungsschritten in einen dreidimensionalen k-Raum einsortiert werden. Es kann dann entsprechend eine Rekonstruktion eines dreidimensionalen Bilddatenvolumens mittels einer dreidimensionalen Fourier-Transformation erfolgen.

Üblicherweise werden zur Ansteuerung eines Magnetresonanztomographiesystems bei der Messung bestimmte vorgegebene Pulssequenzen, d. h. Abfolgen von definierten HF-Pulsen sowie von Gradientenpulsen in verschiedenen Richtungen und von Auslesefenstern, währenddessen die Empfangsantennen auf Empfang geschaltet sind und die Magnetresonanzsignale empfangen und verarbeitet werden, verwendet. Mit Hilfe eines sogenannten Messprotokolls werden diese Sequenzen für eine gewünschte Untersuchung, zum Beispiel einen bestimmten Kontrast der berechneten Bilder, vorab parametrisiert. Das Messprotokoll kann auch weitere Steuerdaten für die Messung enthalten. Dabei gibt es eine Vielzahl von Magnetresonanz-Sequenztechniken, nach denen Pulssequenzen aufgebaut sein können. Eine der großen Herausforderungen an die zukünftige Entwicklung in der Magnetresonanzbildgebung (MR-Bildgebung) ist eine Beschleunigung von Magnetresonanz-Sequenztechniken ohne weitgehende Kompromisse bezüglich Auflösung, Kontrast und Artefaktanfälligkeit.

Die momentane klinische MR Bildgebung beruht fast ausschließlich auf der sogenannten kartesischen (engl. "Cartesian") oder rechtwinkligen (engl. "rectilinear") Bildgebung, bei der die abgetasteten k-Raum-Punkte (d.h. die Abtastpunkte im k-Raum, an denen Rohdaten erfasst werden) auf den Gitterpunkten eines rechtwinkligen Gitters bzw. Rasters liegen. Dabei ist es mit sogenannten parallelen Bildgebungsmethoden gelungen, die klinische MR-Bildgebung signifikant zu beschleunigen. Bei der parallelen MR-Bildgebung PAT wird die Datenakquisition verkürzt, indem ein Teil der zur Rekonstruktion eines einfaltungsfreien Bildes eigentlich notwendigen Zeilen des Rasters im k-Raum nicht akquiriert werden. Diese fehlenden Zeilen werden später während der Bildrekonstruktion im k-Raum substituiert oder es werden die aus der Unterabtastung resultierenden Einfaltungsartefakte im Bildraum entfernt. Eine Voraussetzung, um die parallelen Bildgebungsmethoden einsetzen zu können, ist der Empfang der Hochfrequenzsignale mit mehreren Empfangsspulen (Antennen), wobei die räumliche Empfindlichkeit der einzelnen Empfangsspulen bekannt sein muss. Die räumliche Empfindlichkeit der Empfangsspulen wird mit Hilfe von sogenannten Spulenkalibrierungsdaten berechnet. Die Spulenkalibrierungsdaten müssen in der Regel hinreichend abgetastet sein. Da die Empfindlichkeiten in der Regel räumlich langsam variieren, genügt es in der Regel, wenn die Spulenkalibrierungsdaten räumlich niedrig aufgelöst sind. In der Regel müssen die Spulenkalibrierungsdaten für jeden Patienten neu gemessen werden. Eine der wichtigsten parallelen Bildgebungsmethoden ist das sogenannte GRAPPA-Verfahren, wie es z. B. in dem Artikel "Generalized Autocalibrating Partially Parallel Acquisitions (GRAPPA)" von Marc Griswold et al. in Magnetic Resonance in Medicine 47, 2002, S. 1202 bis 1210, beschrieben wird. Dabei werden die "fehlenden" Rohdaten $s_i(k_y,k_x)$ der Spule i an der k-Raum Position $k=(k_y,k_x)$ mit den k-Raum-Koordinaten $(k_y,k_x)$, an der keine Daten akquiriert wurden, als Linearkombination aller gemessenen Datenpunkte in einer festgelegten Umgebung bzw. Nachbarschaft $\Omega_{(ky,kz)}$ des fehlenden Abtastpunkts berechnet bzw. interpoliert:

$$s_i(k_y,k_x) = \sum_{j=1}^{N_c} \sum_{(q_y,q_x)\in\Omega_{(ky,kx)}} n_{i,(k_y,k_x)}(j,q_y,q_x)s_j(q_y,q_x) \qquad (1)$$

Dabei sind i und j die Laufvariablen für die einzelnen bei der parallelen Messung genutzten Empfangsspulen und laufen jeweils von 1 bis $N_C$, der maximalen Anzahl der genutzten Empfangsspulen. Die äußere (erste) Summe in der Formel (1) zählt über alle Empfangsspulen, die innere (zweite) Summe zählt über alle "gemessenen" Abtastpunkte, an denen Rohdaten akquiriert wurden, und die in eine definierte Nachbarschaft $\Omega_{(ky,kz)}$ des jeweils "fehlenden" Abtastpunktes mit den k-Raum-Koordinaten $(k_y,k_x)$ fallen. $s_j(q_y,q_x)$ ist jeweils das von der j-ten Empfangsspule am Abtastpunkt mit den k-Raum-Koordinaten $(q_y,q_x)$ gemessene Signal (d.h. die dort akquirierten Rohdaten). $n_{i,(ky,kx)}$ sind die komplexen linearen Faktoren die die einzelnen gemessenen Datenpunkte in der Umgebungen $\Omega_{(ky,kz)}$ gewichten und zunächst unbekannt sind. Der Index $\{i, (k_y,k_x)\}$ deutet dabei an, dass man im Allgemeinen nicht nur für jede Spule i sondern auch für jeden nicht gemessenen Datenpunkt mit den Koordinaten $(k_y,k_x)$ einen separaten Satz von Linearfaktoren benötigt.

Ein Kernpunkt dieser Methode ist, dass die Koeffizienten bzw. Gewichtungsfaktoren $n_{i,(ky,kx)}$ (im Folgenden auch "GRAPPA-Gewichte" genannt) in der Formel (1) für rechtwinklige Bildgebung vom Ort $(k_y,k_x)$ des Abtastpunkts im Raster unabhängig sind, sondern nur von den Abständen zu den jeweiligen berücksichtigten Nachbar-Abtastpunkten abhängen:

$$s_i(k_y,k_x) = \sum_{j=1}^{N_c} \sum_{l=0}^{N_y-1} \sum_{m=0}^{N_x-1} n_i(j,l,m)\, s_j\left(k_y + (Al-l_0)\Delta k_y,\; k_x + (m-\frac{N_x}{2})\Delta k_x\right) \qquad (2)$$

Darin ist $\Delta k_y$ der Gitterabstand (das Rastermaß) zwischen benachbarten Abtastpunkten in Phasenkodierrichtung, $\Delta k_x$ ist der Gitterabstand zwischen benachbarten Abtastpunkten in Frequenzkodierrichtung und A ist der Beschleunigungsfaktor. l und m sind Laufvariablen der Nachbar-Abtastpunkte. $l_0$ ist so gewählt, dass alle Abtastpunkte auf der rechten Seite der Gleichung (2) gemessen wurden und Nachbar-Abtastpunkte von $s_i$ sind. $n_i$ sind wiederum die komplexen linearen Faktoren, die die einzelnen gemessenen Datenpunkte in der Umgebung gewichten und zunächst unbekannt sind. In Formel (2) umfasst die rechtwinklige Umgebung eines jeden nichtgemessene Datenpunktes $N_x \times N_y$ gemessene Datenpunkte, von denen jeder mit $N_C$ verschiedenen Komponentenspulen erfasst wurde. Da auf der linken Seite der Gleichung (2) die nicht gemessenen Daten für jede Komponentenspule separat berechnet werden und sich die linearen Faktoren für verschiedene Komponentenspulen unterscheiden, werden insgesamt $N_{unknown} = N_c \cdot N_y \cdot N_x \cdot N_C$ komplexe GRAPPA-Gewichte benötigt, um die nicht gemessenen Daten rekonstruieren zu können. Die GRAPPA-Gewichte erhält man nun dadurch, indem man einen zweiten Datensatz, den sogenannten "Spulenkalibrierungsdatensatz" misst. Dieser Spulenkalibrierungsdatensatz wird vollständig (also hinreichend nach Nyquist) abgetastet bzw. gemessen. Wegen der vollständigen Abtastung sind für den zweiten Datensatz sowohl die Rohdaten $s_i(k_y,k_x)$ auf der linken Seite der Formel (2) wie auch die Rohdaten $s_j(q_y,q_x)$ auf der rechten Seite der Formel (2) bekannt. Besteht der Spulenkalibrierungsdatensatz also aus mindestens sovielen Datenpunkten wie es unbekannte GRAPPA-Gewichte gibt, so können die GRAPPA-Gewichte berechnet werden. Dazu kann die Gleichung (2) am einfachsten für jede Komponentenspule in Matrixform geschrieben werden:

$$\mathbf{s}_i = \mathbf{G} \cdot \mathbf{n}_i \qquad (3)$$

Darin ist $\mathbf{n}_i$ ein Spaltenvektor der Länge $N_y \cdot N_x \cdot N_C$ dessen Komponenten die gesuchten GRAPPA-Gewichte für die Spule i enthalten. Der Spaltenvektor $\mathbf{s}_i$ ist ein Vektor, bestehend aus M Datenpunkten des Spulenkalibrierungsdatensatzes für die auch alle Nachbarn in der gewählten rechtwinkligen Umgebung gemessen wurden. Der Spaltenvektor $\mathbf{s}_i$ hat also die Länge M und enthält nur Datenpunkte der ausgewählten Komponentenspule i. G ist demnach eine M x $N_y \cdot N_x \cdot N_C$ Matrix. Die Elemente der Matrix G bestehen aus gemessenen Datenpunkten. Die m-te Zeile der Matrix G besteht also aus den insgesamt $N_y \cdot N_x \cdot N_C$ Datenpunkten in der rechtwinkligen Umgebung des m-ten Datenpunktes gemäß Gleichung (3).

In der Regel werden so viele Abtastpunkte gemessen, dass das Gleichungssystem überbestimmt ist. Dieses Gleichungssystem wird dann im Sinne der kleinsten quadratischen Abweichung mit Standardmethoden gelöst.

Zusammenfassend lässt sich also sagen, dass eine häufig verwendete Aufnahmetechnik im Rahmen der Magnetresonanztomographie auf der Akquisition von mehreren Aufnahmen mit unterschiedlichem Kontrast und späterer Kombination bzw. entsprechendem Post-Processing besteht. Hierzu zählen zum Beispiel Aufnahmetechniken mit der Bezeichnung MapIT ("MAgnetic Particle Imaging Technology"), DIXON, Diffusion, MP2RAGE ("Magnetization Prepared Two RApid Gradient Echo").

Nachteil des Standes der Technik ist, dass eine Verwendung von GRAPPA zur Reduktion der Aufnahmezeit zu einer nachteilhaften Bildqualität bis hin zu deutlichen Artefakten führt. Das liegt daran, dass um die Bildrekonstruktionszeit zu minimieren, die GRAPPA-Gewichte nur für einen der verschiedenen, aufgenommenen Kontraste berechnet werden. Diese GRAPPA-Gewichte werden dann aber für die Rekonstruktion aller Kontraste verwendet.

Beispielsweise werde für die MP2RAGE-Aufnahmetechnik zwei unterschiedliche IR-Kontraste mit unterschiedlichen Inversionszeiten TI aufgenommen, wobei nach einem Invertierungspuls (IR-Puls), zwei subsequente IR-Kontraste akquiriert werden. Die für PAT benötigten Referenzlinien werden aus dem ersten Kontrast genommen. Da in diesem Fall (d.h. für dieses TI) der erste Kontrast deutlich dunkler und geringer ausfällt (wenig Signal-Rausch-Verhältnis aufgrund der gewählten Inversionszeit), sind die verwendeten Referenzlinien nicht optimal für die PAT-Rekonstruktion des zweiten Kontrastes. Bei Wahl der Referenzlinien aus dem zweiten Kontrast für die Bildrekonstruktion kann dies je nach Wahl der TI-Zeiten für den ersten Kontrast suboptimal sein. Die separate Aufnahme der Referenzlinien für beide Kontraste ist zeitlich suboptimal und kann ebenso schwankende Signal-Rausch-Verhältnisse für die beiden Kontraste liefern.

Neben der kartesischen gewinnt auch in letzter Zeit die radiale Bildgebung zunehmendes Interesse, primär wegen ihrer relativen Unempfindlichkeit gegenüber Bewegung. Bei der radialen Bildgebung erfolgt die Datenakquisition entlang radialer Speichen, die durch das k-Raum-Zentrum gehen. Die relative Unempfindlichkeit gegenüber Bewegung beruht auf der wiederholten Akquisition des zentralen k-Raums. Jedoch ergeben sich bei der oben genannten Aufnahmetechnik auch hier die vorangehend beschriebenen Nachteile.

Es ist eine Aufgabe der vorliegenden Erfindung, ein alternatives, komfortableres Verfahren und eine Vorrichtung zur Rekonstruktion von Kontrasten aus Magnetresonanzaufnahmen mittels einer Parallelen Aufnahme-Technik, sowie eine entsprechende Steuereinrichtung bzw. ein entsprechendes Magnetresonanztomographiesystem anzugeben, mit dem die oben beschriebenen Nachteile vermieden werden.

Diese Aufgabe wird durch ein Verfahren gemäß Patentanspruch 1, eine Vorrichtung gemäß Patentanspruch 10 sowie durch eine Steuereinrichtung gemäß Patentanspruch 12 und ein Magnetresonanztomographiesystem gemäß Patentanspruch 13 gelöst.

Ein erfindungsgemäßes Verfahren dient zur Rekonstruktion von Kontrasten aus Magnetresonanzaufnahmen mittels einer Parallelen Aufnahme-Technik ("PAT") bzw. einer integrierten Parallelen Aufnahme-Technik ("iPAT"), zur Verbesserung der Bildqualität bei Multi-Kontrast-Aufnahmen.

Das erfindungsgemäße Verfahren umfasst die Schritte:

- Bereitstellung/Erstellung von Magnetresonanz-Rohdaten

Dabei werden Magnetresonanz-Rohdaten für das Verfahren erstellt, also von einem Magnetresonanztomographen ("MRT") aufgenommen oder Daten, die vorher aufgenommen worden sind.

Die Magnetresonanz-Rohdaten enthalten Informationen zu mindestens zwei Kontrasten und umfassen Referenzlinien. Damit enthalten die Magnetresonanz-Rohdaten Referenzlinien zu den Kontrasten, also für mindestens zwei dieser Kontraste. Wohlgemerkt können die Magnetresonanz-Rohdaten einzig Daten umfassen, aus denen später die Bilder der Kontraste rekonstruiert werden ("Hauptdaten"), wobei die Referenzlinien dort enthalten sind. Die Magnetresonanz-Rohdaten können aber zusätzlich zu den Hauptdaten auch noch spezielle Referenzdaten zu Referenzlinien zu den aufgenommenen Kontrasten umfassen.

Referenzlinien sind Bereiche im k-Raum, welche zur Berechnung des GRAPPA-Kernels bzw. bei SENSE für die Bestimmung der Spulensensitivitäten genutzt werden. Dies ist in der Regel ein kleiner, zentraler Teil im k-Raum. Typische Größen sind 20 bis 30 Referenzlinien. Die Referenzlinien können z.B. radial oder zeilenweise angeordnet sein.

Ein Beispiel dafür wären MP2RAGE Daten, welche zwei unterschiedliche Kontraste liefern.

- Rekonstruktion von Referenzlinien-Bildern

Diese Referenzlinien-Bilder werden typischerweise mit einer vergleichsweise niedrigeren Auflösung erstellt, um eine schnelle Vorgehensweise zu bewerkstelligen. Die Rekonstruktion erfolgt aus einem Set von Referenzlinien der Magnetresonanz-Rohdaten für mindestens zwei der Kontraste.

Es können dabei in Anlehnung an die oben genannten Beispiele zur Natur der Magnetresonanz-Rohdaten die "Hauptdaten" zu den Kontrasten oder die Referenzdaten zur Rekonstruktion der Referenzlinien-Bilder verwendet werden.

Bei der Rekonstruktion von Referenzlinien-Bildern wird nicht unbedingt eine PAT Rekonstruktion benötigt. Vorteilhaft ist, hierfür, ein schnell durchzuführendes Rekonstruktionsverfahren zu wählen.

- Histogramm-Analyse

Es erfolgt eine Analyse der Referenzlinien-Bilder auf der Basis von Histogrammen. Die Analyse wird dabei so durchgeführt, dass von ihr eine Entscheidung abgeleitet werden kann, welche Referenzlinien für eine PAT-Rekonstruktion verwendet werden sollen.

- PAT-Rekonstruktion

Es erfolgt eine PAT-Rekonstruktion von Bilddarstellungen der unterschiedlichen Kontraste, wobei die Entscheidung, welche Referenzlinien für die PAT-Rekonstruktion verwendet werden, basierend auf der Histogramm-Analyse getroffen wird.

Eine erfindungsgemäße Vorrichtung zur Rekonstruktion von Kontrasten aus Magnetresonanzaufnahmen mit einer Parallelen Aufnahme-Technik, umfasst die folgenden Komponenten:

- Eine Datenschnittstelle zur Erfassung von Magnetresonanz-Rohdaten für mindestens zwei Kontraste, wobei diese Magnetresonanz-Rohdaten Referenzlinien umfassen.

- Eine Referenzlinien-Bild-Rekonstruktionseinheit ausgelegt für eine automatische Rekonstruktion von Referenzlinien-Bildern aus Referenzlinien der Magnetresonanz-Rohdaten für mindestens zwei der Kontraste.

- Eine Analyseeinheit ausgelegt für eine Histogramm-Analyse der Referenzlinien-Bilder.

- Eine Bildrekonstruktionseinheit ausgelegt zur PAT-Rekonstruktion von Bilddarstellungen der unterschiedlichen Kontraste, wobei die Entscheidung, welche Referenzlinien für die PAT-Rekonstruktion verwendet werden, basierend auf der Histogramm-Analyse getroffen wird.

Eine erfindungsgemäße Steuereinrichtung zur Steuerung eines Magnetresonanztomographiesystems ist für die Durchführung eines erfindungsgemäßen Verfahrens ausgelegt und/oder umfasst eine erfindungsgemäße Vorrichtung.

Ein erfindungsgemäßes Magnetresonanztomographiesystem umfasst eine erfindungsgemäße Steuereinrichtung.

Ein Großteil der zuvor genannten Komponenten der Vorrichtung bzw. der Steuereinrichtung, können ganz oder teilweise in Form von Softwaremodulen in einem Prozessor einer entsprechenden Vorrichtung bzw. Steuereinrichtung realisiert werden. Eine weitgehend softwaremäßige Realisierung hat den Vorteil, dass auch schon bisher verwendete Vorrichtungen bzw. Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem bzw. eine Speichereinrichtung einer Steuereinrichtung eines Magnetresonanztomographiesystems ladbar ist, mit Programmabschnitten, um alle Schritte des erfindungsgemäßen Verfahrens auszuführen, wenn das Programm in dem Rechensystem bzw. der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen

Zum Transport zum Rechensystem bzw. zur Steuereinrichtung und/oder zur Speicherung an oder in dem Rechensystem bzw. der Steuereinrichtung kann ein computerlesbares Medium, beispielsweise ein Memorystick, eine Festplatte oder ein sonstiger transportabler oder fest eingebauter Datenträger dienen, auf welchem die von einem Rechensystem bzw. einer Rechnereinheit der Steuereinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bevorzugt wird zur Histogramm-Analyse für mindestens zwei, insbesondere für alle, Referenzlinien-Bilder jeweils ein für jedes Referenzlinien-Bild individuelles Histogramm berechnet. Die Entscheidung, welche Referenzlinien für die PAT-Rekonstruktion verwendet werden, wird dann bevorzugt basierend auf der Signalstärke der in das Histogramm eingeordneten Pixel der Referenzlinien-Bilder getroffen. Bevorzugt werden dabei die Referenzlinien gewählt, deren in das Histogramm eingeordneten Pixel die größte Signalstärke anzeigen. Besonders bevorzugt wird dabei der Schwerpunkt der in dem Histogramm aufgetragenen Verteilung betrachtet und nicht alleine diejenigen Pixel, welche die höchste Signalstärke anzeigen.

Bevorzugt wird bei der Histogramm-Analyse dabei der Mittelwert und die Standardabweichung der Signalverteilung in den Referenzlinien-Bildern, insbesondere in deren Histogrammen, bestimmt.

Bevorzugt erfolgt nach der Rekonstruktion der Referenzlinien-Bilder und vor der Histogramm-Analyse eine Maskierung der Referenzlinien-Bilder und/oder deren Histogramme mittels einer Maske. Beispielsweise werden aus einem maskierten Bild die Anzahl der Pixel innerhalb der Maske ermittelt oder es werden nur diejenigen Pixel innerhalb der Maske in ein Histogramm aufgenommen. Es kann bevorzugt ein Referenzlinien-Bild und/oder ein Histogramm so maskiert werden, dass alle Pixel jenseits einer Grenze (z.B. unterhalb einer Rauschschwelle) nicht mehr betrachtet werden. Dies hat den Vorteil, dass Rauschbereiche für die nachfolgende Analyse ausgeschlossen werden können, da diese nicht zur Entscheidung beitragen können, welche Referenzlinien verwendet werden sollen.

Eine bevorzugte Vorrichtung umfasst eine Maskierungseinheit, ausgelegt für eine Maskierung der Referenzlinien-Bilder und/oder deren Histogrammen.

Die Histogramm-Analyse wird dann bevorzugt basierend auf den maskierten Referenzlinien-Bildern und oder den maskierten Histogrammen durchgeführt.

Die Maskierung findet besonders bevorzugt im Bildraum statt, bevorzugt mit einer Schwellen-Segmentierung. Dabei werden nur Pixel mit einem Wert oberhalb der Schwelle werden in die Maske aufgenommen. Aus den Pixeln innerhalb der Maske wird dann das Histogramm bestimmt. Es sind alternativ oder ergänzend zu einer Schwellen-Segmentierung sind aber auch andere Segementierungen möglich, z.B. RegionGrowing oder aktive Konturen.

Bevorzugt stellen die Referenzlinien-Bilder die Grundlage für die Maskierung dar.

Alternativ erfolgt die Maskierung bevorzugt basierend auf zugehörigen Prescan-Normalize-Daten. Dabei werden zwei Bilder aufgenommen, eines mit der BodyCoil welche als homogen angenommen wird und eines mit den Lokal-Spulen, welche starke Empfangsprofile aufweisen. Aus dem Verhältnis kann dann eine Normalisierungskorrektur für die tatsächliche Aufnahme berechnet werden. Gleichzeitig kann aber auch eine Maske berechnet werden mit der gleichen Segmentierung wie oben beschrieben. Vorteil ist, dass sich die Kontraste und damit der Schwellwert weniger stark ändern, vor allem im Bild der BodyCoil hat man meistens sehr ähnliche Signalstärken.

Bevorzugt werden diejenigen Referenzlinien für die PAT-Rekonstruktion verwendet, bei denen die in das jeweilige Histogramm eingeordneten Pixel die höchste Signalstärke aufweisen. Besonders bevorzugt werden dabei, wie vorangehend beschrieben, nur Pixel innerhalb der Maske betrachtet.

Bevorzugt hängt die Auswahl derjenigen Referenzlinien, die für die PAT-Rekonstruktion verwendet werden, von der Homogenität der Signalverteilung im Histogramm ab und/oder von dem mathematischen Produkt der Anzahl der Pixel in der Maske und dem Mittelwert des Histogramms.

Bevorzugt werden für die PAT-Rekonstruktion aller Kontraste dieselben Referenzlinien verwendet. Es ist aber je nach Anwendung auch bevorzugt, dass zur PAT-Rekonstruktion für unterschiedliche Kontraste unterschiedliche Referenzlinien verwendet werden. Des Weiteren bevorzugt ist eine mathematische Mischung der Referenzlinien beider Kontraste, z.B. ein Mittelwert von Referenzlinie erster und zweiter Kontrast.

Bevorzugt wird als Referenzline eine Line zur Berechnung des GRAPPA-Kernels oder eine Linie für die Bestimmung der Spulensensitvitäten im Rahmen von SENSE gewählt.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:

Figur 1 eine schematische Darstellung eines Verfahrens gemäß dem Stand der Technik,

Figur 2 eine schematische Darstellung des erfindungsgemäßen Verfahrens,

Figur 3 Referenzlinien im k-Raum,

Figur 4 eine Maskierung von Bilddaten,

Figur 5 eine Maskierung von Histogrammdaten,

Figur 6 eine schematische Darstellung einer Histogramm-Analyse,

Figur 7 eine schematische Darstellung eines Magnetresonanztomographiesystems,

Figur 8 eine Vorrichtung gemäß einem Ausführungsbeispiel der Erfindung in Form eines Blockschaltbildes.

In den folgenden Figuren sind nur für die Erfindung wesentliche oder zu ihrem Verständnis hilfreiche Elemente eingezeichnet. So sind z.B. keine Schichtselektionsgradienten dargestellt, obwohl sie in der Pulssequenz durchaus vorhanden sein können.

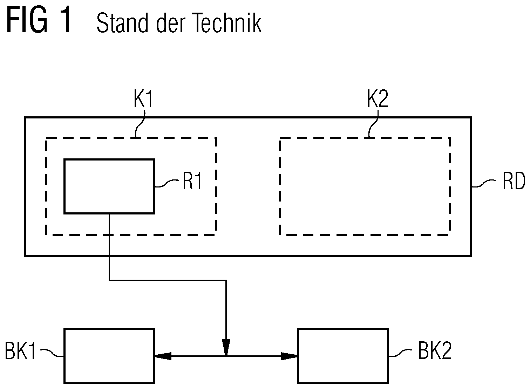

Figur 1 zeigt eine schematische Darstellung eines Verfahrens gemäß dem Stand der Technik. Zuerst erfolgt eine Erstellung von Magnetresonanz-Rohdaten RD für mindestens zwei Kontraste K1, K2, wobei diese Magnetresonanz-Rohdaten RD Referenzlinien R1 umfassen.

Diese Referenzlinien R1 werden zur PAT-Rekonstruktion von Bilddarstellungen BK1, BK2 der unterschiedlichen Kontraste K1, K2 verwendet.

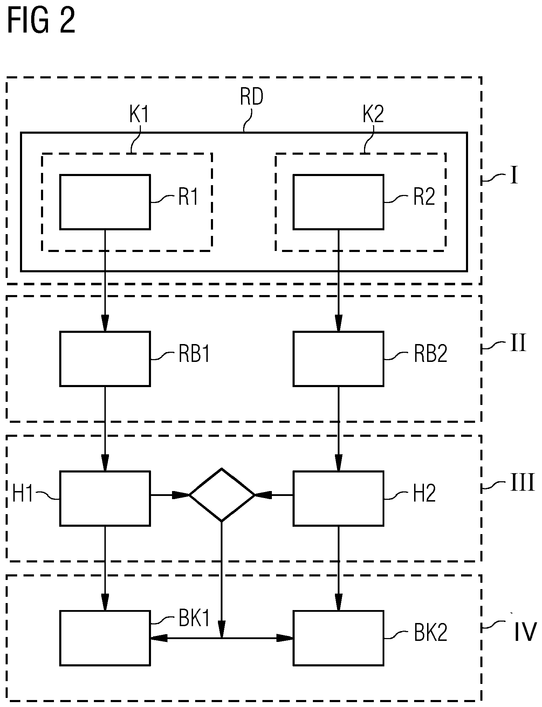

Figur 2 verdeutlicht das erfindungsgemäße Verfahren zur Rekonstruktion von Kontrasten K1, K2 aus Magnetresonanzaufnahmen.

In Schritt I erfolgt eine Bereitstellung (z.B. per PACS) oder Erstellung (mittels eines MRT) von Magnetresonanz-Rohdaten RD für zwei Kontraste K1, K2. Dabei umfassen diese Magnetresonanz-Rohdaten RD, in diesem Fall die Aufnahmen zu den Kontrasten K1, K2, Referenzlinien R1, R2.

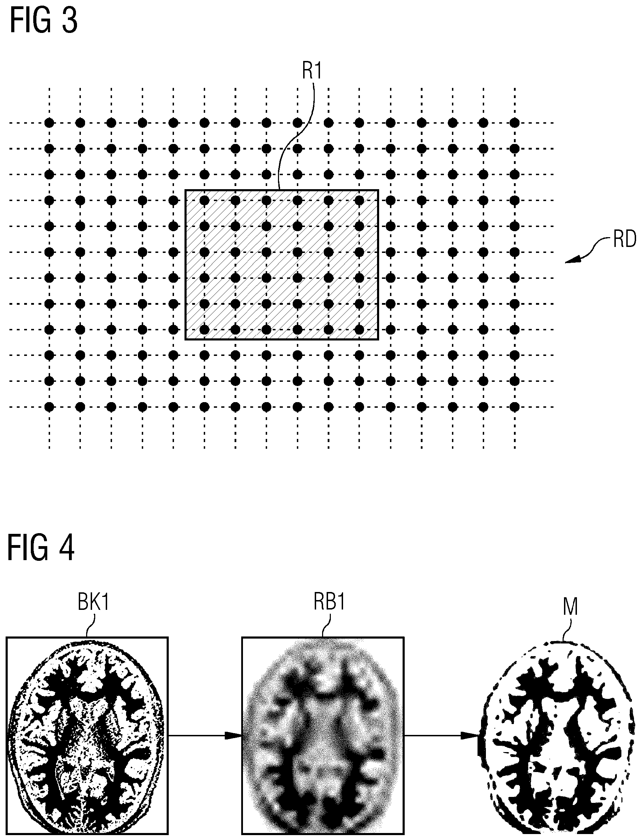

In Schritt II erfolgt eine Rekonstruktion von Referenzlinien-Bildern RB1, RB2 aus Referenzlinien R1, R2 (s. Figur 3) der Magnetresonanz-Rohdaten RD für beide Kontraste K1, K2.

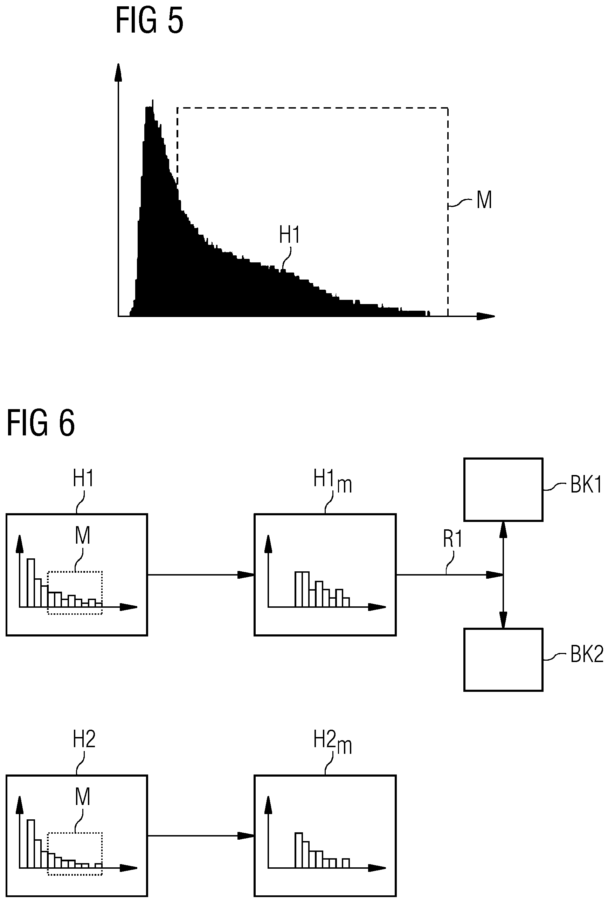

In Schritt III erfolgt eine Histogramm-Analyse basierend auf den Referenzlinien-Bildern RB1, RB2. Dazu wird für jedes Referenzlinien-Bild RB1, RB2 ein Histogramm H1, H2 (s. Figur 5) erstellt und diese Histogramme H1, H2 analysiert (s. Figur 6) .

In Schritt IV erfolgt dann eine PAT-Rekonstruktion von Bilddarstellungen BK1, BK2 der beiden Kontraste K1, K2, wobei die Entscheidung, welche Referenzlinien R1, R2 für die PAT-Rekonstruktion verwendet werden, basierend auf der Histogramm-Analyse getroffen wird.

Figur 3 skizziert Referenzlinien R1 im k-Raum. Die Magnetresonanz-Rohdaten RD, sind hier als eine Matrix äquidistanter Punkte im k-Raum dargestellt. Die Referenzlinien R1 werden nun vorzugsweise aus dem Zentrum des K-Raums genommen, wie es mit dem schraffierten Kasten dargestellt ist. Die Punkte im Inneren dieses Kastens sind die in dem Verfahren bevorzugt verwendeten Referenzlinien.

Figur 4 verdeutlicht das Prinzip einer Maskierung eines Referenz-Bildes RB1 anhand einer Reihe von Bildern. Zunächst wird ein Kontrast aufgenommen (hier bereits als rekonstruiertes Kontrastbild BK1 dargestellt), der aus einem Datensatz von Magnetresonanz-Rohdaten RB und ggf. anderen Daten besteht. Aus diesen Rohdaten wird eine Untermenge von k-Raum-Daten gebildet, welche die Referenzlinien R1 darstellen (s. dazu auch Figur 3). Aus diesen Referenzlinien wird ein Referenzlinien-Bild RB1 rekonstruiert, wie es hier dargestellt ist. Da das Referenzlinien-Bild RB1 nicht den vollen Rohdatensatz des Kontrasts umfasst, ist es auch nicht so detailliert, wie das Kontrastbild KB1.

Das Referenzlinien-Bild RB1 wird nun maskiert, so dass das Rauschen herausgeschnitten wird. Es ergibt sich eine Maske M umfassend diejenigen Bildpunkte, die für die Histogramm-Analyse verwendet werden.

Es ist aber auch eine andere Art der Maskierung möglich.

Figur 5 zeigt eine Maskierung von Histogrammdaten. Aus einem Referenzlinien-Bild RB1, z.B. dem Referenzlinien-Bild RB1 aus Figur 4, wird ein Histogramm gebildet, mit dem Wert eines Bildpunktes auf der x-Achse und der Anzahl von Bildpunkten mit diesem Wert im Referenzlinien-Bild RB1 auf der y-Achse. Um das Rauschen zu unterdrücken, kann der linke Teil des Histogramms H1 abgeschnitten werden, bzw. nur diejenigen Bildpunkte betrachtet werden, die im Histogramm H1 innerhalb der Maske M liegen. Die Einträge in der Maske M im Histogramm H1 würden sich auch ergeben, wenn das Referenzlinien-Bild RB1 entsprechend maskiert worden wäre.

Figur 6 zeigt eine schematische Darstellung einer Histogramm-Analyse. Hier werden zunächst zwei Histogramme H1, H2 aus zwei Referenzlinien-Bildern RB1, RB2 gebildet, wie z.B. vorangehend beschrieben wurde. Die beiden Histogramme H1, H2 werden wie in Figur 5 erklärt maskiert (entweder durch Maskierung der Referenzlinien-Bilder RB1, RB2 oder durch direkte Maskierung der Histogramme H1, H2) und nur die Einträge innerhalb der Maske M betrachtet. Es ergeben sich zwei maskierte Histogramme $H1_m$, $H2_m$. Nun wird in den maskierten Histogrammen $H1_m$, $H2_m$ betrachtet, welches mehr Bildpunkte umfasst (bei vor der Maskierung normierten Histogrammen H1, H2) oder bei welchem Histogramm H1, H2 der Schwerpunkt bei einem höheren Wert liegt. In dem dargestellten Fall ist dies das obere maskierte Histogramm $H1_m$. Die diesbezüglichen Referenzlinien R1 werden dann gemäß dem erfindungsgemäßen Verfahren für eine Unterstützung der Rekonstruktion der Kontrast-Bilder KB1, KB2 ausgewählt.

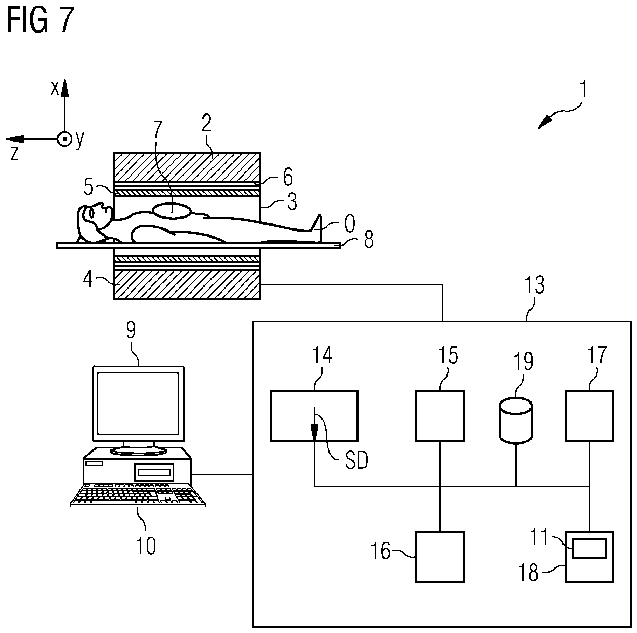

In Figur 7 ist grob schematisch ein Magnetresonanztomographiesystem 1 dargestellt. Es umfasst zum einen den eigentlichen Magnetresonanzscanner 2 mit einem Untersuchungsraum 3 bzw. Patiententunnel, in den auf einer Liege 8 ein Patient oder Proband positioniert ist, in dessen Körper sich das eigentliche Untersuchungsobjekt O befindet. Auch wenn in dem dargestellten Beispiel das Untersuchungsobjekt O im Torso abgebildet ist, wird die Diffusions-Tensor-Bildgebung auch oft für Aufnahmen des Gehirns verwendet, da sie besonders gut zur Abbildung neurologischer Strukturen geeignet ist.

Der Magnetresonanzscanner 2 ist in üblicher Weise mit einem Grundfeldmagnetsystem 4, einem Gradientensystem 6 sowie einem HF-Sendeantennensystem 5 und einem HF-Empfangsantennensystem 7 ausgestattet. In dem dargestellten Ausführungsbeispiel handelt es sich bei dem HF-Sendeantennensystem 5 um eine im Magnetresonanzscanner 2 fest eingebaute Ganzkörperspule, wogegen das HF-Empfangsantennensystem 7 aus am Patienten bzw. Probanden anzuordnenden Lokalspulen besteht (in Figur 7 nur durch eine einzelne Lokalspule symbolisiert). Grundsätzlich können aber auch die Ganzkörperspule als HF-Empfangsantennensystem und die Lokalspulen als HF-Sendeantennensystem genutzt werden, sofern diese Spulen jeweils in unterschiedliche Betriebsweisen umschaltbar sind. Das Grundfeldmagnetsystem 4 ist hier in üblicher Weise so ausgebildet, dass es ein Grundmagnetfeld in Längsrichtung des Patienten erzeugt, d. h. entlang der in z-Richtung verlaufenden Längsachse des Magnetresonanzscanners 2. Das Gradientensystem 6 umfasst in üblicher Weise einzeln ansteuerbare Gradientenspulen, um unabhängig voneinander Gradienten in x-, y- oder z-Richtung schalten zu können. Zudem enthält der Magnetresonanzscanner 2 (nicht dargestellte) Shimspulen, die in üblicher Weise ausgebildet sein können.

Bei dem in Figur 7 dargestellten Magnetresonanztomographie-System handelt es sich um eine Ganzkörperanlage mit einem Patiententunnel, in den ein Patient komplett eingebracht werden kann. Grundsätzlich kann die Erfindung aber auch an anderen Magnetresonanztomographie-Systemen, z. B. mit seitlich offenem, C-förmigen Gehäuse, verwendet werden. Wesentlich ist nur, dass entsprechende Aufnahmen des Untersuchungsobjekts O angefertigt werden können.

Das Magnetresonanztomographie-System 1 weist weiterhin eine zentrale Steuereinrichtung 13 auf, die zur Steuerung des MR-Systems 1 verwendet wird. Diese zentrale Steuereinrichtung 13 umfasst eine Sequenzsteuereinheit 14. Mit dieser wird die Abfolge von Hochfrequenz-Pulsen (HF-Pulsen) und von Gradientenpulsen in Abhängigkeit von einer gewählten Pulssequenz PS oder einer Abfolge von mehreren Pulssequenzen zur Aufnahme mehrerer Schichten in einem interessierenden Volumenbereich des Untersuchungsobjekts innerhalb einer Messsitzung gesteuert. Eine solche Pulssequenz PS kann beispielsweise innerhalb eines Mess- oder Steuerprotokolls P vorgegeben und parametrisiert sein. Üblicherweise sind verschiedene Steuerprotokolle P für unterschiedliche Messungen bzw. Messsitzungen in einem Speicher 19 hinterlegt und können von einem Bediener ausgewählt (und bei Bedarf gegebenenfalls geändert) und dann zur Durchführung der Messung genutzt werden. Im vorliegenden Fall enthält die Steuereinrichtung 13 Pulssequenzen zur Akquisition der Rohdaten.

Zur Ausgabe der einzelnen HF-Pulse einer Pulssequenz PS weist die zentrale Steuereinrichtung 13 eine Hochfrequenzsendeeinrichtung 15 auf, die die HF-Pulse erzeugt, verstärkt und über eine geeignete Schnittstelle (nicht im Detail dargestellt) in das HF-Sendeantennensystem 5 einspeist. Zur Steuerung der Gradientenspulen des Gradientensystems 6, um entsprechend der vorgegebenen Pulssequenz PS die Gradientenpulse passend zu schalten, weist die Steuereinrichtung 13 eine Gradientensystemschnittstelle 16 auf. Die Sequenzsteuereinheit 14 kommuniziert in geeigneter Weise, z. B. durch Aussendung von Sequenzsteuerdaten SD, mit der Hochfrequenzsendeeinrichtung 15 und der Gradientensystemschnittstelle 16 zur Ausführung der Pulssequenz PS.

Die Steuereinrichtung 13 weist außerdem eine (ebenfalls in geeigneter Weise mit der Sequenzsteuereinheit 14 kommunizierende) Hochfrequenzempfangseinrichtung 17 auf, um innerhalb der durch die Pulssequenz PS vorgegebenen Auslesefenster koordiniert mittels des HF-Empfangsantennensystems 7 Magnetresonanz-Signale zu empfangen und so die Rohdaten zu akquirieren.

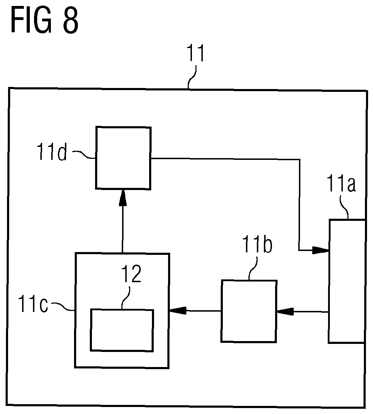

Eine Rekonstruktionseinheit 18 übernimmt hier die akquirierten Rohdaten und rekonstruiert daraus Magnetresonanz-Bilddaten. Diese Rekonstruktionseinheit umfasst eine erfindungsgemäße Vorrichtung 11, (s. Figur 8) die weiter unten genauer erklärt wird.

Wie im Detail durch ein Einstrahlen von HF-Pulsen und das Schalten von Gradientenpulsen geeignete Rohdaten akquiriert und daraus MR-Bilder oder Parameter-Karten rekonstruiert werden können, ist dem Fachmann grundsätzlich bekannt und wird daher hier nicht näher erläutert.

Eine Bedienung der zentralen Steuereinrichtung 13 kann über ein Terminal mit einer Eingabeeinheit 10 und einer Anzeigeeinheit 9 erfolgen, über das somit auch das gesamte Magnetresonanztomographie-System 1 durch eine Bedienperson bedient werden kann.

Das erfindungsgemäße Magnetresonanztomographie-System 1 und insbesondere die Steuereinrichtung 13 können darüber hinaus noch eine Vielzahl von weiteren, hier nicht im Einzelnen dargestellten, aber üblicherweise an derartigen Anlagen vorhandenen Komponenten aufweisen, wie beispielsweise eine Netzwerkschnittstelle, um das gesamte System mit einem Netzwerk zu verbinden und Rohdaten und/oder Bilddaten bzw. Parameter-karten, aber auch weitere Daten, wie beispielsweise patientenrelevante Daten oder Steuerprotokolle, austauschen zu können.

Wie durch ein Einstrahlen von HF-Pulsen und die Erzeugung von Gradientenfeldern geeignete Rohdaten akquiriert und daraus Magnetresonanztomographie-Bilder rekonstruiert werden können, ist dem Fachmann grundsätzlich bekannt und wird hier nicht näher erläutert. Ebenso sind verschiedenste Messsequenzen, wie z. B. EPI-Messsequenzen oder andere Messsequenzen zur Erzeugung von diffusionsgewichteten Bildern, dem Fachmann vom Grundsatz her bekannt.

Figur 8 zeigt ein bevorzugtes Ausführungsbeispiel für eine erfindungsgemäße Vorrichtung 11 in Form eines Blockschaltbildes. Diese Vorrichtung 11 erlaubt der Rekonstruktionseinheit 18 eine vorteilhafte Bildrekonstruktion und wird bevorzugt von der Rekonstruktionseinheit 18 umfasst, kann aber auch ein separates Element, z.B. im Terminal, darstellen. Die Vorrichtung 11 umfasst:

Eine Datenschnittstelle 11a zur Erfassung von Magnetresonanz-Rohdaten RD für mindestens zwei Kontraste K1, K2. In diesem Beispiel können über die Datenschnittstelle 11a auch rekonstruierte Bilddaten übermittelt werden.

Eine Referenzlinien-Bild-Rekonstruktionseinheit 11b, die für eine automatische Rekonstruktion von Referenzlinien-Bildern RB1, RB2 aus Referenzlinien R1, R2 der Magnetresonanz-Rohdaten RD für mindestens zwei der Kontraste K1, K2 ausgelegt ist.

Eine Analyseeinheit 11c, die für eine Histogramm-Analyse der Referenzlinien-Bilder RB1, RB2 ausgelegt ist. Die Analyseeinheit 11c umfasst dabei eine Maskierungseinheit 12, die für eine Maskierung der Referenzlinien-Bilder RB1, RB2 und/oder deren Histogrammen H1, H2 ausgelegt ist.

Eine Bildrekonstruktionseinheit 11d, die zur PAT-Rekonstruktion von Bilddarstellungen der unterschiedlichen Kontraste K1, K2 ausgelegt ist, wobei die Entscheidung, welche Referenzlinien für die PAT-Rekonstruktion verwendet werden, basierend auf der Histogramm-Analyse getroffen wird. Die Bildrekonstruktionseinheit 11d sendet hier die rekonstruierten Kontrastbilder wieder an die Datenschnittstelle 11a.

Es wird abschließend noch einmal darauf hingewiesen, dass es sich bei den vorhergehend detailliert beschriebenen Verfahren sowie bei dem dargestellten Magnetresonanztomographiesystem 1 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Rekonstruktion von Kontrasten (K1, K2) aus Magnetresonanzaufnahmen mittels einer Parallelen Aufnahme-Technik umfassend die Schritte:

   - Bereitstellung oder Erstellung von Magnetresonanz-Rohdaten (RD) für mindestens zwei Kontraste (K1, K2), wobei diese Magnetresonanz-Rohdaten (RD) Referenzlinien (R1, R2) umfassen,
   - Rekonstruktion von Referenzlinien-Bildern (RB1, RB2) aus Referenzlinien (R1, R2) der Magnetresonanz-Rohdaten (RD) für mindestens zwei der Kontraste (K1, K2),
   - Histogramm-Analyse basierend auf den Referenzlinien-Bildern (RB1, RB2),
   - PAT-Rekonstruktion von Bilddarstellungen (BK1, BK2) der unterschiedlichen Kontraste (K1, K2), wobei die Entscheidung, welche Referenzlinien (R1, R2) für die PAT-Rekonstruktion verwendet werden, basierend auf der Histogramm-Analyse getroffen wird.

2. Verfahren nach Anspruch 1, wobei zur Histogramm-Analyse für mindestens zwei Referenzlinien-Bilder (RB1, RB2) jeweils ein für jedes Referenzlinien-Bild (RB1, RB2) individuelles Histogramm (H1, H2) berechnet wird, und die Entscheidung, welche Referenzlinien (R1, R2) für die PAT-Rekonstruktion verwendet werden, bevorzugt basierend auf der Signalstärke der in das Histogramm (H1, H2) eingeordneten Pixel der Referenzlinien-Bilder (RB1, RB2) getroffen wird.

3. Verfahren nach einem der vorangehenden Ansprüche, wobei bei der Histogramm-Analyse der Mittelwert und die Standardabweichung der Signalverteilung in den Referenzlinien-Bildern (RB1, RB2), insbesondere in deren Histogrammen (H1, H2), bestimmt wird.

4. Verfahren nach einem der vorangehenden Ansprüche, wobei vor der Histogramm-Analyse eine Maskierung der Referenzlinien-Bilder (RB1, RB2) und/oder deren Histogramme (H1, H2) mittels einer Maske (M) erfolgt und die Histogramm-Analyse bevorzugt basierend auf den maskierten Referenzlinien-Bildern (RB1, RB2) und/oder den maskierten Histogrammen ($H1_m$, $H2_m$) durchgeführt wird.

5. Verfahren nach Anspruch 4, wobei die Referenzlinien-Bilder (RB1, RB2) die Grundlage für die Maskierung darstellen oder wobei die Maskierung basierend auf zugehörigen Prescan-Normalize-Daten erfolgt.

6. Verfahren nach einem der vorangehenden Ansprüche, wobei diejenigen Referenzlinien (R1, R2) für die PAT-Rekonstruktion verwendet werden, bei denen die in das jeweilige Histogramm (H1, H2) eingeordneten Pixel die höchste Signalstärke aufweisen.

7. Verfahren nach einem der vorangehenden Ansprüche, wobei die Auswahl derjenigen Referenzlinien (R1, R2), die für die PAT-Rekonstruktion verwendet werden, von der Homogenität der Signalverteilung im Histogramm (H1, H2) abhängt und/oder von dem mathematischen Produkt der Anzahl der Pixel in der Maske (M) und dem Mittelwert des Histogramms (H1, H2) abhängt.

8. Verfahren nach einem der vorangehenden Ansprüche, wobei für die PAT-Rekonstruktion aller Kontraste (K1, K2) dieselben Referenzlinien (R1, R2) verwendet werden oder für unterschiedliche Kontraste (K1, K2) unterschiedliche Referenzlinien (R1, R2) verwendet werden.

9. Verfahren nach einem der vorangehenden Ansprüche, wobei als Referenzline (R1, R2) eine Line zur Berechnung des GRAPPA-Kernels oder eine Linie für die Bestimmung der Spulensensitvitäten im Rahmen von SENSE gewählt wird.

10. Vorrichtung (11) zur Rekonstruktion von Kontrasten (K1, K2) aus Magnetresonanzaufnahmen mit einer Parallelen Aufnahme-Technik, umfassend

- eine Datenschnittstelle (11a) zur Erfassung von Magnetresonanz-Rohdaten (RD) für mindestens zwei Kontraste (K1, K2), wobei diese Magnetresonanz-Rohdaten (RD) Referenzlinien (R1, R2) umfassen,
- eine Referenzlinien-Bild-Rekonstruktionseinheit (11b) ausgelegt für eine automatische Rekonstruktion von Referenzlinien-Bildern (RB1, RB2) aus Referenzlinien (R1, R2) der Magnetresonanz-Rohdaten (RD) für mindestens zwei der Kontraste (K1, K2),
- eine Analyseeinheit (11c) ausgelegt für eine Histogramm-Analyse der Referenzlinien-Bilder (RB1, RB2),
- eine Bildrekonstruktionseinheit (11d) ausgelegt zur PAT-Rekonstruktion von Bilddarstellungen der unterschiedlichen Kontraste (K1, K2), wobei die Entscheidung, welche Referenzlinien für die PAT-Rekonstruktion verwendet werden, basierend auf der Histogramm-Analyse getroffen wird.

11. Vorrichtung (11) nach Anspruch 10, umfassend eine Maskierungseinheit (12), ausgelegt für eine Maskierung der Referenzlinien-Bilder (RB1, RB2) und/oder deren Histogrammen (H1, H2) .

12. Steuereinrichtung (13) zur Steuerung eines Magnetresonanztomographiesystems (1), welche zur Durchführung eines Verfahrens nach einem der Ansprüche 1 bis 9 ausgestaltet ist und/oder eine Vorrichtung nach Anspruch 10 oder 11 umfasst.

13. Magnetresonanztomographiesystem (1) umfassend eine Steuereinrichtung (13) nach Anspruch 12.

14. Computerprogrammprodukt mit einem Computerprogramm, welches direkt in eine Speichereinrichtung eines Rechensystems oder einer Steuereinrichtung (13) eines medizintechnischen bildgebenden Systems (1) ladbar ist, mit Programmabschnitten, um alle Schritte des Verfahrens nach einem der Ansprüche 1 bis 9 auszuführen, wenn das Computerprogramm in dem Rechensystem oder der Steuereinrichtung (13) ausgeführt wird.

15. Computerlesbares Medium, auf welchem von einer Rechnereinheit einlesbare und ausführbare Programmabschnitte gespeichert sind, um alle Schritte eines Verfahrens nach einem der Ansprüche 1 bis 9 auszuführen, wenn die Programmabschnitte von der Rechnereinheit ausgeführt werden.

**Claims**

1. Method for reconstructing contrast levels (K1, K2) from magnetic resonance acquisitions by means of a parallel acquisition technique comprising the steps of:

- providing or generating magnetic resonance raw data (RD) for at least two contrast levels (K1, K2), wherein this magnetic resonance raw data (RD) comprises reference lines (R1, R2),
- reconstructing reference line images (RB1, RB2) from reference lines (R1, R2) of the magnetic resonance raw data (RD) for at least two of the contrast levels (K1, K2),
- carrying out a histogram analysis on the basis of the reference line images (RB1, RB2),
- carrying out a PAT reconstruction of image representations (BK1, BK2) of the different contrast levels (K1, K2), wherein the decision as to which reference lines (R1, R2) are used for the PAT reconstruction is made on the basis of the histogram analysis.

2. Method according to claim 1, wherein, for the purposes of histogram analysis, an individual histogram (H1, H2) for each reference line image (RB1, RB2) is in each case calculated for at least two reference line images (RB1, RB2) and the decision as to which reference lines (R1, R2) are used for the PAT reconstruction is preferably made on the basis of the signal strength of the pixels, classified in the histogram (H1, H2), of the reference lines (RB1, RB2).

3. Method according to one of the preceding claims, wherein the mean and the standard deviation of the signal distribution in the reference line images (RB1, RB2), in particular in the histograms (H1, H2) thereof, are determined in the histogram analysis.

4. Method according to one of the preceding claims, wherein, before histogram analysis, the reference line images (RB1, RB2) and/or the histograms (H1, H2) thereof are masked by means of a mask (M) and the histogram analysis is preferably carried out on the basis of the masked reference line images (RB1, RB2) and/or the masked histograms ($H1_m$, $H2_m$).

5. Method according to claim 4, wherein the reference line images (RB1, RB2) are the basis for the masking or wherein masking proceeds on the basis of associated Prescan Normalize data.

6. Method according to one of the preceding claims, wherein the reference lines (R1, R2) used for the PAT reconstruction are those in which the pixels classified in the respective histogram (H1, H2) have the highest signal strength.

7. Method according to one of the preceding claims, wherein the selection of those reference lines (R1, R2) which are used for the PAT reconstruction is dependent on the homogeneity of the signal distribution in the histogram (H1, H2) and/or on the mathematical product of the number of pixels in the mask (M) and the mean of the histogram (H1, H2).

8. Method according to one of the preceding claims, wherein the same reference lines (R1, R2) are used for the PAT reconstruction of all the contrast levels (K1, K2) or different reference lines (R1, R2) are used for different contrast levels (K1, K2).

9. Method according to one of the preceding claims, wherein one line for calculating the GRAPPA kernel or one line for determining coil sensitivities for the purposes of SENSE is selected as the reference line (R1, R2).

10. Apparatus (11) for reconstructing contrast levels (K1, K2) from magnetic resonance acquisitions with a parallel acquisition technique comprising

- a data interface (11a) for acquiring magnetic resonance raw data (RD) for at least two contrast levels (K1, K2), wherein this magnetic resonance raw data (RD) comprises reference lines (R1, R2),
- a reference line image reconstruction unit (11b) designed for automatic reconstruction of reference line images (RB1, RB2) from reference lines (R1, R2) of the magnetic resonance raw data (RD) for at least two of the contrast levels (K1, K2),
- an analysis unit (11c) designed for histogram analysis of the reference line images (RB1, RB2),
- an image reconstruction unit (11d) designed for PAT reconstruction of image representations of the different contrast levels (K1, K2), wherein the decision as to which reference lines are used for the PAT reconstruction is made on the basis of the histogram analysis.

11. Apparatus (11) according to claim 10, comprising a masking unit (12) designed for masking the reference line images (RB1, RB2) and/or the histograms (H1, H2) thereof.

12. Control device (13) for controlling a magnetic resonance tomography system (1), which device is designed for carrying out the method according to one of claims 1 to 9 and/or comprises the apparatus according to claim 10 or 11.

13. Magnetic resonance tomography system (1) comprising a control device (13) according to claim 12.

14. Computer program product with a computer program which is directly loadable into a storage device of a computer system or a control device (13) of a medical imaging system (1), with program parts for carrying out all the steps of the method according to one of claims 1 to 9 when the computer program is executed in the computer system or control device (13).

**15.** Computer-readable medium on which are stored the program parts which can be read in and executed by a computer unit in order to carry out all the steps of the method according to one of claims 1 to 9 when the program parts are executed by the computer unit.

**Revendications**

**1.** Procédé de reconstruction de contrastes (K1, K2) à partir d'enregistrements de résonnance magnétique au moyen d'une technique d'enregistrement parallèle, comprenant les stades :

- on se procure ou on établit des données (RD) brutes de résonnance magnétique pour au moins deux contrastes (K1, K2), ces données (RD) brutes de résonnance magnétique comprenant des lignes (R1, R2) de référence,
- on reconstruit des images (RB1, RB2) de lignes de référence à partir des lignes (R1, R2) de référence des données (RD) brutes de résonnance magnétique pour au moins deux des contrastes (K1, K2),
- on effectue une analyse d'histogramme reposant sur les images (RB1, RB2) de lignes de référence,
- on effectue une reconstruction PAT de représentation (BK1, BK2) d'image des contrastes (K1, K2) différents, la décision des lignes (R1, R2) de référence à utiliser pour la reconstruction PAT étant prise sur la base de l'analyse d'histogramme.

**2.** Procédé suivant la revendication 1, dans lequel pour l'analyse d'histogramme, on calcule pour au moins deux images (RB1, RB2) de ligne de référence respectivement un histogramme (H1, H2) individuel pour chaque image (RB1, RB2) de lignes de référence et la décision des lignes (R1, R2) de référence à utiliser pour la reconstruction PAT est prise de préférence en se basant sur l'intensité de signal des pixels, mis dans l'histogramme (H1, H2), des images (RB1, RB2) de lignes de référence.

**3.** Procédé suivant l'une des revendications précédentes, dans lequel dans l'analyse d'histogramme, on détermine la moyenne et l'écart-type de la distribution de signal dans les images (RB1, RB2) de lignes de référence, notamment dans leurs histogrammes (H1, H2).

**4.** Procédé suivant l'une des revendications précédentes, dans lequel, avant l'analyse d'histogramme, on effectue un masquage des images (RB1, RB2) de lignes de référence et/ou de leurs histogrammes (H1, H2) au moyen d'un masque (M)
et on effectue l'analyse d'histogramme de préférence en se basant sur les images (RB1, RB2) de lignes de référence masquées et/ou sur les histogrammes ($H1_m$, $H2_m$) masqués.

**5.** Procédé suivant la revendication 4, dans lequel les images (RB1, RB2) de lignes de référence représentent le fondement du masquage ou dans lequel le masquage s'effectue sur la base de données prescan-normalize associées.

**6.** Procédé suivant l'une des revendications précédentes, dans lequel on utilise pour la reconstruction PAT les lignes (R1, R2) de référence pour lesquelles les pixels mis dans l'histogramme (H1, H2) respectif ont l'intensité du signal la plus grande.

**7.** Procédé suivant l'une des revendications précédentes, dans lequel la sélection des lignes (R1, R2) de référence, qui sont utilisées pour la reconstruction PAT, dépend de l'homogénéité de la distribution du signal dans l'histogramme (H1, H2) et/ou du produit mathématique du nombre de pixels dans le masque (M) par la moyenne de l'histogramme (H1, H2).

**8.** Procédé suivant l'une des revendications précédentes, dans lequel on utilise pour la reconstruction PAT tous les contrastes (K1, K2) des mêmes lignes (R1, R2) de référence ou on utilise des lignes (R1, R2) de référence différentes pour des contrastes (K1, K2) différents.

**9.** Procédé suivant l'une des revendications précédentes, dans lequel on choisit comme ligne (R1, R2) de référence une ligne pour le calcul du noyau GRAPPA ou une ligne pour la détermination de la sensibilité de bobine dans le cadre de SENSE.

**10.** Installation (11) de reconstruction de contrastes (K1, K2) à partir d'enregistrements de résonnance magnétique par une technique d'enregistrement parallèle, comprenant

- une interface (11a) de données pour la saisie de données (RD) brutes de résonnance magnétique pour au moins deux contrastes (K1, K2), ces données (RD) brutes de résonnance magnétique comprenant des lignes (R1, R2) de référence,
- une unité (11b) de reconstruction d'image de lignes de référence conçue pour une reconstruction automatique d'image (RB1, RB2) de lignes de référence à partir de lignes (R1, R2) de référence des données (RD) brutes de résonnance magnétique pour au moins deux des contrastes (K1, K2),
- une unité (11c) d'analyse conçue pour une analyse d'histogramme des images (RB1, RB2) de lignes de référence,
- une unité (11d) de reconstruction d'image conçue pour la reconstruction PAT de représentation d'image des contrastes (K1, K2) différents, la décision des lignes de référence à utiliser pour la reconstruction PAT étant prise sur la base de l'analyse d'histogramme.

11. Installation (11) suivant la revendication 10, comprenant une unité (12) de masquage conçue pour un masquage des images (RB1, RB2) et/ou de leurs histogrammes (H1, H2).

12. Dispositif (13) de commande pour la commande d'un système (1) de tomodensitographe à résonnance magnétique, qui est conformé pour effectuer un procédé suivant l'une des revendications 1 à 9 et/ou qui comprend une installation suivant la revendication 10 ou 11.

13. Système (1) de tomodensitogramme à résonnance magnétique comprenant un dispositif (13) de commande suivant la revendication 12.

14. Produit de programme d'ordinateur ayant un programme d'ordinateur, qui peut être chargé directement dans un dispositif de mémoire d'un système informatique ou d'un dispositif (13) de commande d'un système (1) d'imagerie de la technique médicale, comprenant des parties de programme pour effectuer tous les stades du procédé suivant l'une des revendications 1 à 9, lorsque le programme d'ordinateur est réalisé dans le système informatique ou dans le dispositif (13) de commande.

15. Support déchiffrable par ordinateur, sur lequel sont mises en mémoire des parties de programme pouvant être déchiffrées et réalisées par une unité informatique, pour effectuer tous les stades d'un procédé suivant l'une des revendications 1 à 9, lorsque les parties de programme sont réalisées par l'unité informatique.

# FIG 1  Stand der Technik

# FIG 2

FIG 3

FIG 4

## FIG 5

## FIG 6

FIG 7

FIG 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **VON MARC GRISWOLD et al.** Generalized Autocalibrating Partially Parallel Acquisitions (GRAPPA). *Magnetic Resonance in Medicine,* 2002, vol. 47, 1202-1210